# DEMANDE DE BREVET EUROPEEN

(11) **EP 3 825 396 A1**
(43) Date de publication de la demande: **26.05.2021**
(21) Numéro de dépôt: 20209096.5
(22) Date de dépôt: 20.11.2020
(51) Int. Cl.: C12M 3/00, C12M 1/00

(54) **SYSTÈME ET MÉTHODE DE CULTURE DE SOUCHES DE PHYTOPLANCTON**

(30) Priorité: 22.11.2019 FR 1913128
(71) Demandeur: IFREMER, 29280 Plouzane (FR)
(72) Inventeur: BERARD, Jean-Baptiste, 44400 REZE (FR); BOUGARAN, Gaël, 44300 NANTES (FR); ROIG, Nicolas, 44300 NANTES (FR); CARRIER, Gregory, 44700 ORVAULT (FR)
(74) Mandataire: Corret, Hélène

(57) **Abrégé**

Il s'agit d'un système de culture de phytoplancton comprenant au moins un portoir (3) disposé linéairement le long d'une traverse (2), chaque portoir comprenant au moins n (avec n ≥2) photo-bioréacteurs (PBRs) verticaux de rétention d'un mélange aqueux contenant les phytoplanctons et séparés respectivement entre eux et/ou par rapport au portoir adjacent par des moyens de cloisonnement optique. Le système comporte un dispositif d'éclairement comprenant une source radiative s'étendant horizontalement en vis-à-vis des PBRs, ladite source émettant une radiation électromagnétique sur une zone d'homogénéité de façon à ce que la variation de la puissance radiative soit inférieure à un seuil déterminé, et des moyens de contrôle de la puissance radiative émise par la source.

## Description

La présente invention concerne un système de culture de microorganismes et plus particulièrement de culture de souches de phytoplancton permettant de les caractériser et de les comparer entre elles de manière susceptible d'être répétée de façon facile et fiable.

Elle concerne aussi une méthode d'utilisation d'un système de culture de phytoplanctons.

Elle trouve une application particulièrement importante bien que non exclusive dans le domaine de l'étude expérimentale des microalgues ou des cyano-bactéries.

L'un des problèmes posés par de telles cultures réside dans la difficulté de cultiver des souches de façon identique, ou sensiblement identique, pour permettre de réaliser sur ces cultures des mesures de phénotypage, c'est-à-dire permettant la caractérisation comparative de leurs traits phénotypiques (croissance, protéines, glucides, lipides, ...) répondant aux critères de répétitivité et de qualité scientifique, notamment nécessaires à l'exploitation scientifique et industrielle de tels résultats.

La présente invention vise notamment à résoudre ce problème et plus généralement les problèmes issus de l'hétérogénéité des conditions de culture.

Par hétérogénéité, il faut entendre une hétérogénéité des conditions de croissance entre cultures multiples menées en parallèle.

Mais il faut également, entendre une hétérogénéité des conditions de culture et de répétabilité sur une même culture, analysée cette fois-ci avec un décalage dans le temps, de façon à observer notamment l'existence d'une éventuelle dérive.

On connaît déjà dans le domaine des plantes et/ou des phytoplanctons, des dispositifs de culture comprenant des enceintes thermostatiques équipées de lumières permettant des expérimentations.

Plus précisément, les cultures sont ici placées dans des enceintes, à distance de tubes fluorescents tout en réalisant leur homogénéité par bullage.

De tels dispositifs n'assurent pas réellement l'homogénéité spatiale et temporelle de l'éclairement. Ils sont de plus encombrants, ce qui limite le nombre de cultures observables, et ne permettent pas d'accueillir une instrumentation de contrôle/ régulation correcte pour des photo-bioréacteurs.

On connaît aussi (KR 2015 0016771 - CN103396938) des ensembles de photo bioréacteurs alignés autonomes. Mais de tels ensembles n'autorisent en réalité qu'une supervision partagée. Il n'y a aucune garantie d'homogénéité entre chaque bioréacteur et ce d'autant plus que le problème des interférences entre réacteurs adjacents n'avait pas été identifié et/ou résolu précédemment, bien au contraire.

La présente invention vise à fournir un système de culture de phytoplancton répondant mieux que ceux antérieurement connus aux exigences de la pratique, notamment en ce qu'elle permet une excellente homogénéité des conditions de mesure et dès lors des mesures elles-mêmes, sur une seule culture dans le temps et/ou sur plusieurs cultures en même temps, de façon compacte, répétitive, aisée par rapport aux dispositifs classiquement utilisés et pour un coût amélioré. Elle permet de plus la mise en œuvre et l'observation simultanée et/ou dans le temps de plusieurs cultures expérimentales de phytoplancton, en conditions contrôlées, homogène spatialement et temporellement.

En particulier l'invention permet d'éviter les interférences entre cultures adjacentes, selon que les bioréacteurs développent une culture plus sombre ou plus claire que le(s) réacteur(s) adjacent(s), et/ou que ces derniers ne soient vides ou remplis.

En d'autres termes l'irradiation lumineuse reçue par la culture d'un PBR va d'une part être identique ou sensiblement identique à celles reçues par les cultures (s'il y en a) des PBRs adjacents, et/ou d'autre part ne pas être perturbée par les caractéristiques (vide ou plein) ou l'évolution des caractéristiques d'opacité de chacune de ces cultures au cours du temps, ce qui pourrait générer une absorption/réflexion parasite de la quantité de lumière reçue par un PBR du fait de la modification des caractéristiques d'opacité du ou des PBRs adjacent(s). Ainsi, grâce à un tel système et à la mise en place et au suivi des conditions opératoires de ces cultures avec un tel système il est possible de façon efficace et répétable d'obtenir d'excellentes conditions de mesure, et dès lors une mesure des résultats fiable et reproductible.

Dans ce but la présente invention propose notamment un système de culture de phytoplancton comprenant : une traverse horizontale sur laquelle est monté au moins un portoir disposé linéairement le long de ladite traverse,
le ou les portoirs comprenant chacun au moins n (avec n ≥2) photo-bioréacteurs (PBRs) verticaux de rétention d'un mélange aqueux contenant ledit phytoplancton
le système comportant en outre un dispositif d'éclairement comprenant une source radiative électromagnétique comportant au moins un bandeau radiatif s'étendant horizontalement en vis-à-vis des PBRs,
ladite source étant propre à émettre une radiation électromagnétique sur une zone d'homogénéité de longueur D en vis-à-vis et en débordement par rapport au(x) portoir(s), caractérisé en ce que
le ou les portoirs sont montés sur la traverse de façon amovible,
en ce que les PBRs sont séparés respectivement entre eux et/ou par rapport au portoir adjacent, par des moyens de cloisonnement optique,
en ce que la longueur D de la zone d'homogénéité de la source radiative (9) est agencée de façon à ce que la variation de la puissance radiative de ladite source sur cette longueur D soit inférieure à un seuil déterminé,
et en ce qu'il comporte des moyens de contrôle de la puissance radiative émise par ladite source.
De tels moyens de contrôle vont notamment permettre d'obtenir un bon contrôle de la stabilité de ladite puissance radiative, qui peut ainsi être préservée.

Par débordement par rapport au(x) portoir(s) il faut entendre s'étendant longitudinalement en dépassement latéral de part et d'autre des extrémités latérales du portoir ou de l'ensemble de portoirs.

Par s'étendant en vis-à-vis des PBRs, il faut entendre en vis-à-vis des flacons ou enceintes des PBRs, et plus particulièrement des parties des enceintes de ces derniers agencés pour contenir le mélange aqueux à mesurer.

Les moyens de cloisonnement (par exemple opaques et/ou réfléchissants) permettent la séparation optique entre les PBRs, et sont avantageusement agencés pour assurer une bonne circulation de l'air thermostatique environnant.

En précisant que la longueur D est agencée pour limiter la variation de puissance radiative à une valeur seuil déterminée, le système comportant par ailleurs des moyens de contrôle (et donc de stabilisation)de ladite puissance radiative, l'homme du métier va pouvoir dimensionner structurellement son système, sans pour autant se limiter à des valeurs de dimensionnement spécifiques de manière injustifiée.

Plus précisément, en fonction de la puissance de la source lumineuse (nombre de bandeaux radiatifs, emplacements et puissance de ces derniers), du nombre de PBRs et de leur dimensionnement (volume de rétention de la culture, hauteur, forme - par exemple cylindrique et tronconique en partie basse), de l'écartement entre PBRs dans le sens longitudinal du portoir et d'une simple campagne d'essai à vide et/ou avec un liquide d'étalonnage (de l'eau par exemple) pour tarer le dispositif, l'homme du métier va déterminer la longueur D, en fonction du seuil déterminé (par exemple +/-5%) qu'il veut respecter, tout en contrôlant la puissance radiative et sa stabilité par les moyens de commande prévus au système.

Avantageusement le système comporte au moins deux portoirs alignés.

Avantageusement le système comprend pour chaque PBR, des moyens de mesure passive de densité optique (DO) montés de l'autre côté de chaque PBR par rapport à la source, agencés pour mesurer la puissance de la radiation détectée au travers du mélange aqueux comprenant le phytoplancton, et des moyens d'homogénéisation par bullage du dit mélange aqueux.

Les portoirs, et le dimensionnement de la traverse, sont agencés pour que les doses d'éclairage émises par la source et reçues par chaque portoir soient équivalentes, et que la variabilité horizontale de la quantité et/ou densité de lumière reçue par chaque PBR soit minimisée, par exemple inférieure ou égale à 5%.

À noter que l'homogénéité dans le sens vertical est quant à elle identique et/ou équivalente entre tous les PBRs par construction.

Avantageusement il existe au moins deux bandeaux radiatifs, par exemple trois ou quatre bandeaux identiques, lesdits bandeaux étant par exemple rectangulaires, parallèles disposés horizontalement.

Dans un mode de réalisation avantageux, les bandeaux sont formés par des alignements de LEDs de puissance, de sorte que, par exemple, on obtienne une puissance de 25W par mètre linéaire.

La lumière obtenue avec l'ensemble des bandeaux radiatifs présente ainsi des caractéristiques très fiables et contrôlables.

Il est observé ici que la lumière est un paramètre particulièrement difficile à contrôler spatialement et temporellement sur une multiplicité de flacons de PBR.

Pour ce faire, il a en général été prévu par le passé, une source verticale par PBR (contrairement à l'invention).

Également avantageusement il est prévu au moins un capteur optique de contrôle complémentaire disposé en vis-à-vis du dispositif d'éclairement.

Avantageusement, il existe un capteur par portoir. Ces capteurs permettent de mesurer l'intensité de l'éclairement incident et d'opérer un contrôle de celui-ci en cas d'écart par rapport à la consigne.

Dans des modes de réalisation avantageux, on a de plus et/ou par ailleurs recours à l'une et/ou à l'autre des dispositions suivantes :
- les portoirs sont agencés pour accueillir au moins, 4 PBRs, par exemple 6,8 ou 12 PBRs, c'est-à-dire qu'il comporte au moins quatre, six, huit ou douze cellules de soutien de PBR correspondant ;
- les portoirs sont nettoyables(stérilisables) par autoclave, par exemple fabriqués en acier inoxydable ;
- le flacon de culture (composant le volume de culture du PBR) présente une épaisseur optique qui n'excède pas 5 cm, ce qui permet de limiter les phénomènes d'auto-ombrages cellulaires dans le cas de cultures à forte densité cellulaire ;
- le flacon de culture comprend une restriction du trajet optique dans le mélange aqueux, par exemple inférieur à une longueur de 2cm, par exemple ≤ 1cm, permettant la lecture passive de densité optique. Avantageusement cette restriction est située en partie basse du PBR ;
- les moyens de mesure de la densité optique sont agencés pour mesurer dans une gamme de valeurs, s'inscrivant dans les spectres visible et/ou infrarouge ;
- le dispositif d'éclairement est équipé d'un panneau diffuseur en vis-à-vis des PBRs, ce qui améliore l'homogénéité de la lumière, et diminue les effets de concentration de lumières (effets « spot ») en cas de systèmes LEDs ;
- Le système comporte un automate agencé pour permettre la fixation d'une consigne, et/ou pour réaliser des cycles lumineux dynamiques, par exemple dont les signaux peuvent être de forme carré, sinusoïdale et/ou triangulaire et/ou nycthéméraux ;
- les moyens d'homogénéisation par bullage comprennent un débitmètre à flotteur et/ou un débitmètre massique. Le flux d'air est avantageusement injecté en partie basse du PBR, de manière à supprimer toute possibilité de décantation en fond des PBR ;
- le système comprend des moyens de régulation du pH de chaque PBR. Avantageusement ces moyens de régulation comprennent une boucle de contrôle qui asservit une injection de CO₂ dans le PBR via une électrovanne ;
- le système, configuré pour des cultures en chemostats, comprend pour chaque PBR, une surverse du contenu des flacons et une pompe d'alimentation d'éléments nutritifs du PBR correspondant ;
- le système, configuré pour des cultures en turbidostat, comprend pour chaque PBR une pompe d'alimentation d'éléments nutritifs asservie à une consigne de DO (Densité Optique) attribuée au PBR ;
- le dispositif d'éclairement est équipé en LEDs dites blanches et/ou de couleur(s) ;
- les moyens de mesure des densités optiques traversant les PBRs d'un même portoir sont intégrés à un même élément ;
- la lecture de la densité optique est activée par allumage de LED émettrices en vis-à-vis, par exemple situées dans des loges d'un élément fixe solidaire du portoir concerné de l'autre côté des PBRs ;
- le volume du flacon de chaque PBR est compris entre 300 et 600 ml, avantageusement 500 ml, (autorisant un échantillonnage fréquent des cultures et des analyses biochimiques et moléculaires) ;
- le seuil déterminé de variation de puissance radiative est inférieur ou égal à 10%, par exemple 5% et avantageusement 3% ;
- la source radiative électromagnétique émet dans les longueurs d'ondes correspondant au spectre visible, à l'infrarouge et/ou à l'ultra-violet.

L'invention concerne également une méthode de culture de phytoplancton utilisant ou mettant en œuvre un système tel que décrit ci-avant.

L'invention sera mieux comprise à la lecture de la description qui suit de modes de réalisation donnés ci-après à titre d'exemples non limitatifs. La description se réfère aux dessins qui l'accompagnent dans lesquels :
La figure 1 est une vue schématique en perspective d'un système selon un mode de réalisation de l'invention.
La figure 2 est une vue en perspective d'un portoir avec six PBRs, appartenant à un système selon un mode de réalisation de l'invention.
La figure 3 est une vue schématique en perspective montrant un agencement d'émetteur et de capteur optique de mesure de DO selon un mode de réalisation de l'invention.
Les figures 4 et 4A montrent le principe utilisé pour obtenir une bonne répartition de la puissance radiative lumineuse.
La figure 5 est un schéma montrant un exemple de placement des cloisonnements optiques selon un mode de réalisation de l'invention.

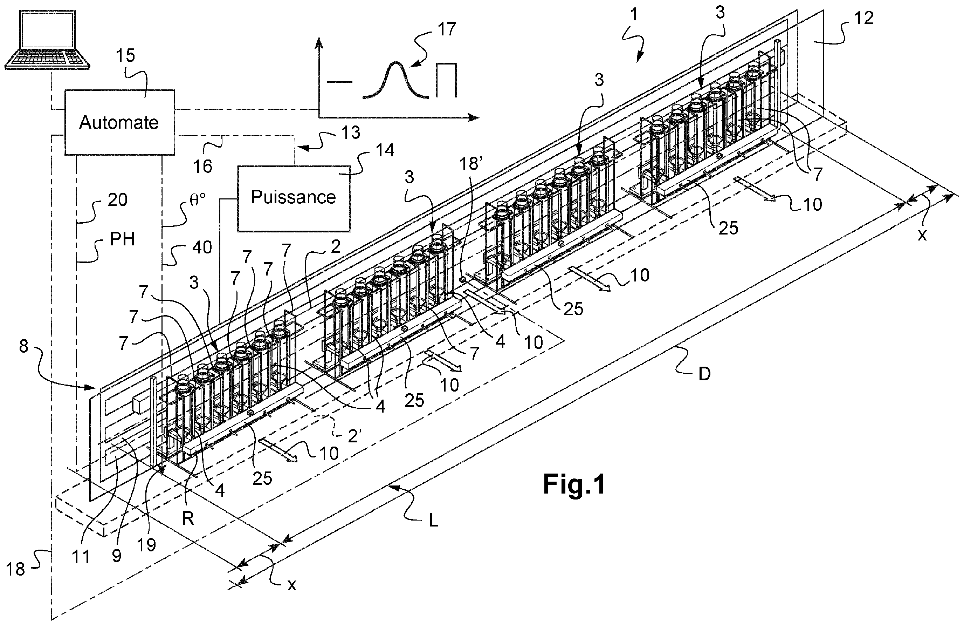

La figure 1 montre un système 1 de culture de phytoplancton comprenant une traverse 2 horizontale formant un bâti monté sur un support (non représenté) par exemple posé sur une surface de table ou de paillasse.

Sur la figure 1 on a représenté la traverse comme une tige ou barre transversale rectangulaire située en partie haute du système en dehors du trajet optique.

Mais la traverse peut être formée par plusieurs éléments rigides 25 fixés solidairement sur un socle 2' au plan de travail 2 en partie basse du système.

Ces éléments fixés ont alors deux fonctions, celle d'une traverse structurelle permettant aux portoirs (voir ci-après) d'être à équidistance de la source de lumière et celle de support éventuel de moyens optiques inférieurs, tels que décrits ci-après.

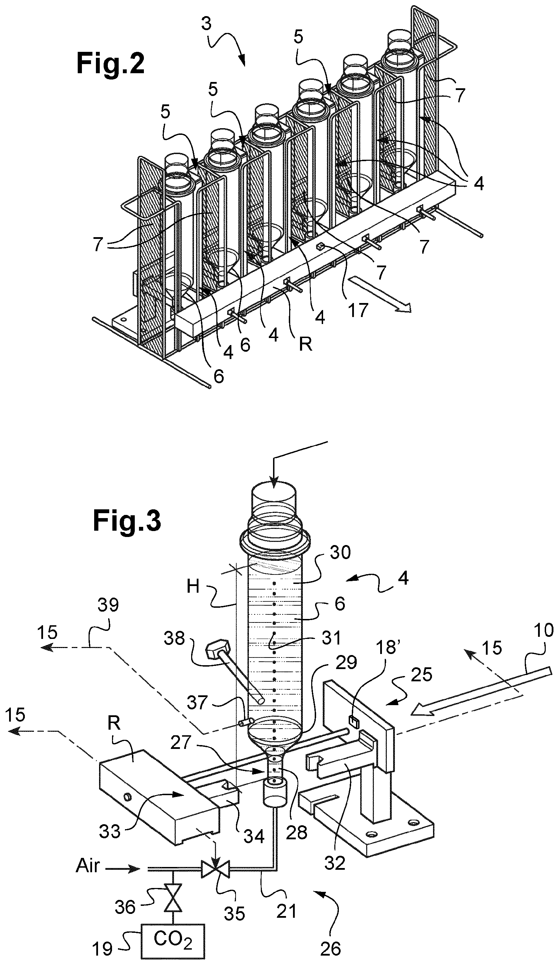

Quatre portoirs identiques 3 (voir également figure 2), pouvant chacun contenir six photo bioréacteurs ou PBR 4 de rétention des mélanges aqueux contenant les micro algues du phytoplancton, sont fixés de façon amovible et connue en elle-même, sur la traverse, de façon répartie linéairement.

Les PBRs sont chacun respectivement placés dans des alvéoles ou cellules 5 de support des flacons ou enceintes 6 des bioréacteurs 4 qui seront décrits de façon plus détaillée en référence à la figure 3.

Chaque flacon comprend le mélange à mesurer qui est séparé du flacon adjacent par une cloison opaque 7, représentée en transparence sur les figures pour les rendre plus lisibles, par exemple formé par une plaque rectangulaire verticale en matériau opaque à la lumière sur la hauteur active, de flacon contenant le volume de mélange à étudier.

Le système 1 comprend un dispositif 8 d'éclairement comprenant une source radiative 9 de lumière (électromagnétique) qui diffuse la lumière (flèche 10) au travers des PBR.

Dans le mode de réalisation décrit ici, la source radiative 9 comporte trois bandeaux 11 horizontaux de LEDs de puissance, par exemple des LEDs ayant une température de couleur de 2000°K à 3000°K de puissance de 25 à 50W par mètre linéaire, disposées en nappe les unes à côtés des autres, par exemple à 2 cm l'une de l'autre. Les bandeaux forment par exemple des rectangles allongés de dix centimètres de largeur, espacés les uns des autres de 5 à 10 cm. Mais ils peuvent également constituer une seule surface émettrice, par exemple de 50 cm de largeur.

Un écran diffuseur 12 par exemple formé par une plaque de diffusion en PMMA par exemple d'angle de diffusion de 80° est interposé entre la source radiative 9 et les PBRs 4 pour homogénéiser encore le flux lumineux.

Les bandeaux s'étendent longitudinalement sur une longueur horizontale L créant une zone d'homogénéité de lumière de longueur D en vis-à-vis et en débordement sur une distance x par exemple de 10 cm, de part et d'autre par rapport aux extrémités latérales 12 des portoirs d'extrémité.

Le système comprend des moyens 13 de contrôle de la puissance radiative émise par la source. Les moyens 13 comprennent une alimentation 14 de puissance de la source commandée par un automate 15 connu en lui-même et programmé pour contrôler (lien 16) (consigne fixe ou modulée 17) la lumière 10 émise par la source.

Dans le mode de réalisation décrit ici un rétro contrôle (lien 18) de la consigne s'effectue par le biais de capteurs, par exemple une photodiode 18' disposée de façon centrale au niveau du bas des portoirs, entre la source de lumière et les portoirs, par exemple avec une régulation du type PID.

Des moyens 19 (non représentés sur la figure 1) de régulation du PH (de chaque PBR) connus en eux même, à partir d'une consigne affichée sur l'automate (par exemple pH =7,5) sont prévues (lien 20). Pour ce faire un ajustage se fait par exemple en délivrant ou non du CO2 pendant un certain laps de temps au travers du circuit de gaz 21 par lequel est alimenté un bullage d'air de brassage du mélange (voir figure 3). On observe un excellent respect de la consigne même si, du fait des perturbations dues au bullage, les mesures peuvent ponctuellement descendre en dessous.

Avantageusement le système 1 comprend des moyens (non représentés) connus en eux même, de mesure de la température du contenu (liquide) de chacun des PBRs situés dans la pièce ou salle climatisée dans laquelle est disposé le système, à une température déterminée.

Pour une consigne à 23°C, on observe par exemple un écart type de 0,2°C avec une moyenne de 22,6°C.

Le système 1 comprend de plus, pour chaque PBR, des moyens 25 ou R de mesure passive de la densité optique (DO)de chaque PBR, disposés à intervalle régulier, en face de la partie inférieure 27 de chaque flacon 6 (voir ci-après figures 2 et 3).

Des moyens 26 de bullage sont par ailleurs prévus pour assurer l'homogénéité du mélange aqueux observé.

Plus précisément la figure 3 montre schématiquement et partiellement un PBR 4 utilisé dans le mode de réalisation de l'invention plus particulièrement décrit ici. Chaque flacon 6 comprend une partie inférieure 27 en forme de portion 28 cylindrique rétrécie, ou restriction, de diamètre par exemple 1 cm, en prolongation du fond 29 en forme de cône du flacon 6.

Le flacon 6 est en forme d'éprouvette cylindrique, par exemple de 5 cm de diamètre, dans laquelle le mélange aqueux avec phytoplancton 30 est cultivé sur une hauteur H déterminée, par exemple 30 cm pour 500 ml et dans lequel les bulles 31 issues de l'alimentation en air 21, montent verticalement au travers de la partie cylindrique 28. Plus précisément les moyens 25 de mesures de densité optique comprennent un élément 32, comprenant une diode émettrice de lumière de caractéristique déterminée, au travers de ladite partie inférieure 27, et des moyens capteurs 33 comprenant une photodiode 34 de mesure.

Mais il est également possible d'ajourer en son centre l'élément 32 pour laisser passer la lumière de la source 10 et ainsi d'effectuer une mesure passive grâce au capteur 34.

Dans le mode réalisation plus particulièrement décrit ici les photodiodes 34 de mesure sont intégrées dans une même réglette R qui permet un démontage facile et un alignement reproductible de la mesure optique de façon simple et efficace. Cette réglette permet d'épouser chaque section de mesure grâce à un système d'auto-centrage par exemple formé par des petites alvéoles semi-cylindriques qui coopèrent à frottement avec l'extrémité cylindrique 28 des PBRs servant aux mesures.

À intervalles réguliers, le système (ou banc de phénotypage) procède à une lecture de la densité optique du mélange de chaque PBR.

Pour ce faire, l'automate 15 active des électrovannes 35 en amont pour couper tout apport de gaz dans les PBRs (afin d'éliminer les perturbations liées aux bulles 31 qui remontent le long de la culture).

Après une temporisation suffisante pour s'assurer d'une lecture optimale, l'automate mesure les valeurs de densité optique via les diodes réceptrices 34, puis réactive les électrovannes.

Des moyens (un piquage 37) sur les flacons sont prévus pour effectuer des prélèvements et/ou pour effectuer une mesure de PH 39 sur chaque PBR.

De même un piquage 38 par exemple orienté à 80° par rapport à l'horizontale, permet d'introduire un capteur de température.

Cette mesure est retransmise à l'automate (lien 20-voir figure 1) en continu.

Grâce à l'invention, on obtient une qualité et une répétitivité des mesures de DO exceptionnelles, en particulier on observe une forte corrélation des mesures de DO du système avec un suivi manuel (R² > 0,95).

Pendant les mesures, l'utilisateur peut superviser en continu, l'évolution de la croissance des cultures via un ordinateur 41 programmé pour afficher les données par exemple, sous forme de graphes, les données mesurées étant par ailleurs stockées pour être ultérieurement exploitées.

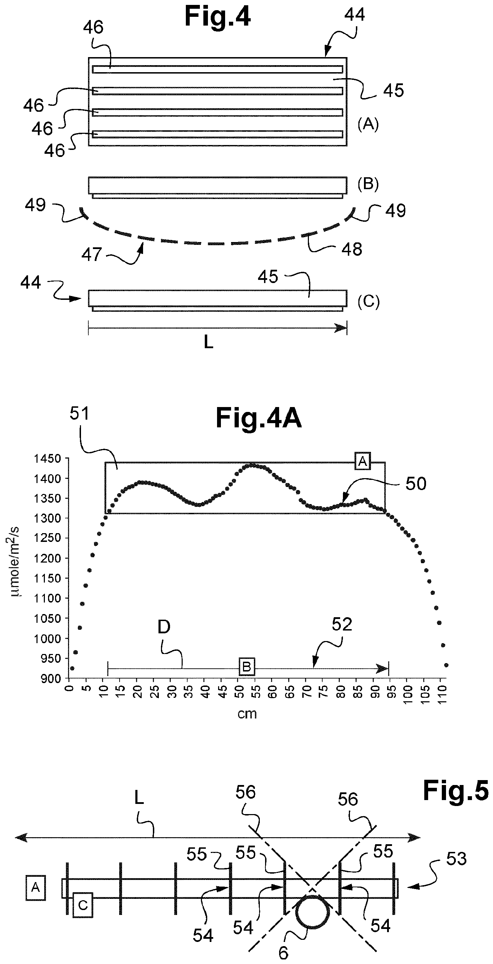

On a représenté sur les figures 4 et 4A, le principe mis en œuvre pour obtenir la bonne homogénéité de la composante lumière de l'invention.

Sur un dispositif 44 ou dalle d'éclairement 45 composée de plusieurs bandeaux 46 de LEDs repartis horizontalement, on recherche une section de puissance de radiation homogène à ± 5%.

Les LEDs émettant selon un cône de lumière, les bandeaux peuvent être espacés transversalement. Tout en permettant d'irradier sur la totalité du PBR en vis-à-vis.

Plus précisément, le dispositif 44 comprend une dalle d'éclairement 45 composée de quatre bandeaux ou rubans 46 de LEDs repartis horizontalement.

La représentation spatiale 47 horizontale d'une même valeur radiative est quant à elle représentée par la courbe en trait interrompu 48. On y distingue des effets de bords 49. Pour déterminer la section recherchée, on réalise une série de mesures de radiation lumineuse sur l'intégralité de la longueur L du dispositif, toutes les mesures étant équidistantes de ce dernier.

On obtient alors une courbe 50 (voir figure 4A) de points de mesure, qui permet de déterminer une zone 51 de valeurs, à partir de laquelle on choisit la section d'homogénéité 52 de longueur D.

Cette zone 52 peut être calculée à partir de plusieurs méthodes, par exemple à partir de la valeur médiane des mesures et en fixant une tolérance de ± 5% de déviation par rapport à cette médiane, ou encore en calculant, la valeur moyenne des mesures après exclusions des valeurs extrêmes des effets de bords, etc.

La figure 5 illustre un positionnement des cloisonnements des PBRs.

Afin de garantir un effet de doses radiatives équivalentes entre chaque PBR, le portoir 53 dont la longueur n'excède pas la longueur D de la section d'homogénéité prévoit des cloisons 54 opaques, par exemple en acier inoxydable, entre chaque PBR.

Pour cela des ailettes 55 rectangulaires disposées sur toute la hauteur des flacons et espacées régulièrement entre chaque cellule de PBR, sont prévues.

Le placement de ces ailettes est calculé à partir des rayons limites optiques 56. La division des ailettes est alors réduite de façon à limiter le confinement thermique des PBRs comme montré graphiquement sur la figure 5.

On va maintenant décrire le fonctionnement ou la mise en œuvre du système de culture de phytoplancton selon le mode de réalisation plus particulièrement décrit ici.

La première étape consiste à préparer les PBRs (flacons, filtres, raccords, bouchons etc.) et à les fixer/disposer sur les portoirs.

L'étape suivante est celle de l'étalonnage de l'instrumentation, et notamment des sondes pH.

Puis on remplit les PBRs avec de l'eau de mer ou de l'eau douce (selon les espèces étudiées, c'est-à-dire marines ou dulçaquicoles).

S'ensuit l'étape de fermeture des flacons.

Vient ensuite l'étape de stérilisation des PBRs.

Pour ce faire le(s) portoir(s) est, (sont) passé(s) au four autoclavable connu en lui-même pendant le temps nécessaire (par exemple 120°C à 2 bars absolu pendant 20 mn). Puis l'ensemble est ramené à la température ambiante.

L'étape suivante est l'étape d'ensemencement.

Le(s) milieu(x) de culture concentré(s) est (sont) tout d'abord introduit (s) dans les PBRs, puis on insère le (ou les) inoculum(a) de phytoplancton à étudier.

Les instruments étant connectés à l'automate 15 et au reste du dispositif permettant le suivi des paramètres de fonctionnement du système, le logiciel de supervision programmé dans l'automate est lancé.

Les débits d'air sont réglés, et les premières mesures pH, températures et luminosité sont effectuées.

Puis on réalise les mesures en fonctionnement nominal selon le(s) cahier(s) des charges d'expérimentation préétablis.

Pour ce faire et après réglage du fonctionnement dynamique du banc et allumage du dispositif d'éclairement on réalise en continu les mesures en fonction du ou des protocoles définis préalablement. Ceux-ci vont pouvoir s'étendre sur plusieurs heures et/ou journées avec une même qualité de reproduction des mesures de DO, PH et températures.

Les instruments connectés à l'automate permettent ainsi d'excellentes supervision et régulation, la quantité de lumière telle qu'apparaissant sur les figures 4 et 4A étant maintenue homogène sur les PBRs, permettant ainsi la construction d'un historique des données exploitable.

On peut ainsi réaliser avec une excellente répétabilité et une grande fiabilité des études inter-espèces, inter-souches, inters lignés, intra-espèce, intra-souche, dans des conditions homogènes et reproductibles entre chaque culture.

Comme il va de soi et comme il résulte également de ce qui précède, la présente invention n'est pas limitée aux modes de réalisation plus particulièrement décrits. Elle en embrasse au contraire toutes les variantes et notamment celles permettant de faire varier les PH, celles permettant les études en cascades (couplage de plusieurs flacons ensemble), les études d'expositions radiatives (UV, visible, IR) ou encore les applications à d'autres modèles planctoniques (bactéries, micromycètes, zooplancton, gamétophytes ...)

## Revendications

1. Système (1) de culture de phytoplancton comprenant : une traverse (2) horizontale sur laquelle est monté au moins un portoir (3) disposé linéairement le long de ladite traverse,
le ou les portoirs comprenant chacun au moins n (avec n ≥2) photo-bioréacteurs (PBRs) (4) verticaux de rétention d'un mélange aqueux contenant ledit phytoplancton ,
le système comportant en outre un dispositif (8) d'éclairement comprenant une source radiative (9) électromagnétique comportant au moins un bandeau (11) radiatif s'étendant horizontalement en vis-à-vis des PBRs,
ladite source étant propre à émettre une radiation électromagnétique (10) sur une zone d'homogénéité de longueur D en vis-à-vis et en débordement par rapport au(x) portoir(s) (3), **caractérisé en ce que** le ou les portoirs sont montés sur la traverse de façon amovible,
**en ce que** les PBRs sont séparés respectivement entre eux et/ou par rapport au portoir adjacent, par des moyens (7) de cloisonnement optique,
**en ce que** la longueur D de la zone d'homogénéité de la source radiative (9) est agencée de façon à ce que la variation de la puissance radiative de ladite source sur cette longueur D soit inférieure à un seuil déterminé,
et **en ce qu'**il comporte des moyens (15) de contrôle de la puissance radiative émise par ladite source.

2. Système (1) de culture selon la revendication 1, **caractérisé en ce qu'**il comprend, pour chaque PBR (4), des moyens (25) de mesure passive de densité optique (DO) montés de l'autre côté de chaque PBR par rapport à la source, agencés pour mesurer la puissance de la radiation détectée au travers du mélange aqueux comprenant le phytoplancton, et des moyens (26) d'homogénéisation par bullage du dit mélange aqueux.

3. Système (1) selon l'une quelconque des revendications précédentes, **caractérisé en ce que** les portoirs (3), et le dimensionnement de la traverse (2), sont agencés pour que les doses d'éclairage émises par la source et reçues par chaque portoir soient équivalentes, et que la variabilité horizontale de la quantité et/ou densité de lumière reçue par chaque PBR soit ≤ 5 %.

4. Système (1) selon l'une quelconque des revendications précédentes, **caractérisé, en ce qu'**il comporte au moins deux bandeaux (11) radiatifs, identiques, rectangulaires, parallèles disposés horizontalement.

5. Système (1) selon l'une quelconque des revendications précédentes, **caractérisé, en ce que** le bandeau (11) est formé par des alignements de LEDs de puissance.

6. Système (1) selon l'une quelconque des revendications précédentes, **caractérisé en ce qu'**il comporte au moins un capteur (17) optique, de contrôle complémentaire disposé en vis-à-vis du dispositif d'éclairement.

7. Système (1) selon l'une quelconque des revendications précédentes, **caractérisé en ce que** chaque portoir (3) comprend au moins quatre cellules (5) pour accueillir au moins 4 PBRs (4).

8. Système (1) selon l'une quelconque des revendications précédentes, **caractérisé en ce que** chaque PBR comportant un flacon (6) de culture, il comprend une restriction (28) du trajet optique dans le mélange aqueux inférieur à une longueur de 2 cm, située en partie basse du PBR (4).

9. Système (1) selon l'une quelconque des revendications précédentes, **caractérisé en ce que** le dispositif d'éclairement est équipé d'un panneau diffuseur (12).

10. Système (1) selon l'une quelconque des revendications précédentes, **caractérisé en ce qu'**il comporte un automate (15) agencé pour permettre la fixation d'une consigne, et/ou pour réaliser des cycles lumineux dynamiques et/ou asservissant une injection de CO₂ dans le PBR (4) via une électrovanne pour contrôle du PH.

11. Système (1) selon l'une quelconque des revendications précédentes, **caractérisé en ce qu'**il est configuré pour des cultures en chemostats et comprend, pour chaque PBR (4), une surverse du contenu des flacons et une pompe d'alimentation d'éléments nutritifs du PBR correspondant.

12. Système (1) selon l'une quelconque des revendications précédentes, **caractérisé en ce qu'**il est configuré pour des cultures en turbidostat et comprend, pour chaque PBR (4), une pompe d'alimentation d'éléments nutritifs asservie à une consigne de DO (Densité Optique) attribué au PBR (4).
